(19) Europäisches Patentamt
European Patent Office
Office européen des brevets

(11) Veröffentlichungsnummer: **0 394 843 B1**

(12) **EUROPÄISCHE PATENTSCHRIFT**

(45) Veröffentlichungstag der Patentschrift: **15.12.93**

(51) Int. Cl.5: **C07D 233/60**, C07D 249/08, C07D 401/06, C07D 405/06, A01N 43/50, A01N 43/653

(21) Anmeldenummer: **90107439.3**

(22) Anmeldetag: **19.04.90**

# (54) 1-Halogenvinyl-azole und diese enthaltende Fungizide und Wachstumsregulatoren.

(30) Priorität: **26.04.89 DE 3913725**

(43) Veröffentlichungstag der Anmeldung:
**31.10.90 Patentblatt 90/44**

(45) Bekanntmachung des Hinweises auf die Patenterteilung:
**15.12.93 Patentblatt 93/50**

(84) Benannte Vertragsstaaten:
**AT BE CH DE DK ES FR GB GR IT LI NL SE**

(56) Entgegenhaltungen:
**EP-A- 0 023 286**
**US-A- 4 273 776**

(73) Patentinhaber: **BASF Aktiengesellschaft**
**Carl-Bosch-Strasse 38**
**D-67063 Ludwigshafen(DE)**

(72) Erfinder: **Seele, Rainer, Dr.**
**Leiblstrasse 3**
**D-6701 Fussgoenheim(DE)**
Erfinder: **Kober, Reiner, Dr.**
**Im Schlittweg 20**
**D-6701 Fussgoenheim(DE)**
Erfinder: **Goetz, Norbert, Dr.**
**Schoefferstrasse 25**
**D-6520 Worms 1(DE)**
Erfinder: **Saupe, Thomas, Dr.**
**Kressenwiesenweg 13**
**D-6902 Sandhausen(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**
Erfinder: **Rademacher, Wilhem, Dr.**
**Austrasse 1**
**D-6703 Limburgerhof(DE)**
Erfinder: **Jung, Johann, Dr. Prof.**
**Hardenburgstrasse 19**
**D-6703 Limburgerhof(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue Azolverbindungen, Verfahren zu ihrer Herstellung und diese enthaltende Fungizide und Wachstumsregulatoren.

Es ist bekannt, Vinylazole z.B. das 1-(1,2,4-Triazol-1-yl)-2-(4-chlorbenzyl)-3-phenyl-propen-1-ol-3 oder das 1-(1,2,4-Triazol-1-yl)-2-(4-chlorphenyl)-3-(2,4-dichlorphenyl)-propen-1-ol-3 als Fungizide zu verwenden (EP 23 286). Ihre Wirkung ist jedoch nicht befriedigend.

Es wurde nun gefunden, daß 1-Halogen-1-Vinylazole der Formel I

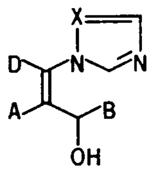

I

in welcher

A und B gleich oder verschieden sind und $C_1$-$C_8$-Alkyl, $C_5$-$C_8$-Cycloalkyl, $C_5$-$C_8$-Cycloalkenyl, Tetrahydropyranyl, Pyridyl, Naphthyl, Biphenyl oder Phenyl bedeutet, wobei diese Reste (1 bis 3 mal) durch Halogen, Nitro, Phenoxy, Amino, $C_1$-$C_4$-Alkyl, $C_1$-$C_4$-Alkoxy oder Halogen-$C_1$-$C_4$-alkyl substituiert sein können,

D den Rest Chlor oder Brom bedeutet,

X den Rest CH oder N bedeutet,

sowie deren für Pflanzen verträgliche Säureadditionssalze oder Metallkomplexe eine bessere fungizide Wirkung besitzen als bekannte Azolverbindungen und eine gute Wirkung als Wachstumsregulatoren besitzen.

Die Verbindungen der Formel I enthalten asymmetrische C-Atome und können daher als Enantiomere und Diastereomere auftreten. Die Gemische von Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Die Racemate lassen sich bei den erfindungsgemäßen Verbindungen nach bekannten Methoden, beispielsweise durch Salzbildung mit einer optisch aktiven Säure, Trennung der diastereomeren Salze und Freisetzung der Enantiomeren mittels einer Base trennen. Als fungizide und und wachstumsregulatorische Mittel kann man die einheitlichen Diastereomere bzw. Enantiomere als auch deren bei der Synthese anfallenden Gemische verwenden.

Bevorzugt werden Verbindungen, in denen A und B die Phenylgruppe bedeutet, welche unsubstituiert oder durch einen oder zwei Substituenten substituiert ist, die Fluor, Chlor, Brom oder die Trifluormethylgruppe bedeuten.

Die Wirkstoffe werden angewendet, indem man eine fungizid wirksame Menge eines 1-Halogen-Vinylazols der Formel I wie oben definiert oder dessen für Pflanzen verträgliches Säureadditionssalz oder Metallkomplex auf die Pilze oder durch Pilzbefall bedrohte Materialien, Flächen, Pflanzen oder Saatgüter einwirken läßt, oder indem man eine regulierend wirksame Menge eines 1-Halogen-Vinylazols der Formel I wie oben definiert oder dessen für Pflanzen vertraglichei Säureadditionssalz oder Metallkomplex auf Kulturpflanzen oder den Boden einwirken läßt.

A bzw. B bedeuten beispielsweise Methyl, Ethyl, Isopropyl, n-Propyl, n-Butyl, sec-Butyl, tert.-Butyl, n-Pentyl, Neopentyl, 1-Naphthyl, 2-Naphthyl, p-Biphenyl, Phenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, 2-Methoxyphenyl, 3-Methoxyphenyl, 4-Methoxyphenyl, 2,4-Dimethoxyphenyl, 4-Ethylphenyl, 4-Isopropylphenyl, 4-tert.-Butylphenyl, 4-tert. Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Aminophenyl, 2-Trifluormethylphenyl, 3-Trifluormethylphenyl, 4-Trifluormethylphenyl, 3-Pyridyl, Tetrahydropyranyl, Cyclopropyl, Cyclopentyl, Cyclohexyl, 2-Cyclohexenyl, 3-Cyclohexenyl.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der 1-Halogen-1-Vinylazole (I) mit geeigneten Säuren.

Metallkomplexe der Wirkstoffe I oder ihrer Salze können mit z.B. Kupfer, Zink, Zinn, Mangan, Eisen, Kobalt oder Nickel gebildet werden, indem man die 1-Halogen-Vinylazole mit entsprechenden Metallsalzen

umsetzt.

Die Verbindungen der Formel I können hergestellt werden, indem man eine Verbindung der Formel II

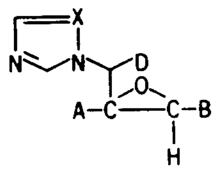

II

in welcher A,B, D und X die oben angegebene Bedeutung haben, in Gegenwart einer Base in die 1-Halogenvinylazole umlagert.

Die Reaktion erfolgt gegebenenfalls in Gegenwart eines Lösungs- oder Verdünnungsmittels unter Zusatz einer anorganischen oder organischen Base bei Temperaturen zwischen 10 und 120 ° C.

Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ether wie Tetrahydrofuran, Diethylether, Dimethoxyethan, Dioxan oder Diisopropylether, Amide wie Dimethylformamid, Dimethylacetamid oder N-Methylpyrrolidon, Sulfolan oder entsprechende Gemische.

Geeignete Basen sind beispielsweise Alkalihydroxide wie Lithium, Natrium- oder Kaliumhydroxid, Alkalihydride wie Lithium-, Natrium- und Kaliumhydrid, Alkaliamide wie Natrium- und Kaliumamid, Alkalicarbonate wie Natrium, Kalium- oder Caesiumcarbonat oder Natrium-, Kalium- oder Caesiumhydrogencarbonat, ferner Natrium- oder Kalium-tert. butoxid, sowie Natrium- oder Kaliummethanolat.

Die Umsetzung wird im allgemeinen bei Temperaturen zwischen 20 und 150 ° C, drucklos oder unter Druck, kontinuierlich oder diskontinuierlich durchgeführt.

Die Ausgangsverbindungen II können hergestellt werden, indem man eine Verbindung der Formel III

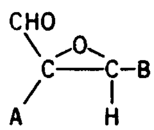

III

in welcher A und B die oben angegebene Bedeutung haben, mit einer Verbindung der Formel IV

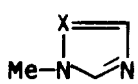

IV

in der X die oben angegebene Bedeutung hat, und Me ein Wasserstoffatom oder ein Metallatom (z.B. Na, K) bedeutet, in Gegenwart der entsprechenden Thionylhalogenide zur Umsetzung bringt.

Die Umsetzung erfolgt gegebenenfalls in Gegenwart eines Lösungs- oder Verdünnungsmittels bei Temperaturen zwischen -30 und 80 ° C. Zu den bevorzugten Lösungs- und Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die neuen Ausgangsverbindungen III erhält man durch Epoxidierung der entsprechenden Olefine V

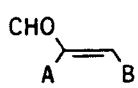

V

in denen A und B die oben angegebenen Bedeutungen haben, mit Peroxycarbonsäuren wie Perbenzoesäure, 3-Chlorperbenzoesäure, 4-Nitroperbenzoesäure, Monoperphthalsäure, Peressigsäure, Perpropionsäure,

Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat oder Triton B. Man arbeitet zwischen 10 und 100 °C und katalysiert die Reaktion gegebenenfalls z.B. mit Jod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30 °C sowie Alkylhydroperoxide, z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Die Verbindungen V lassen sich entsprechend allgemein bekannter Verfahren zur Aldehydsynthese (Houben-Weyl-Müller, Methoden der Organischen Chemie, Georg Thieme Verlag, Stuttgart 1983/4, Bd E 3) herstellen.

Die folgenden Beispiele erläutern die Herstellung der Wirkstoffe.

1. Herstellung der Ausgangsstoffe

Vorschrift 1

E/Z-2-(4-Fluorphenyl)-3-(2-chlorphenyl)-propenal

Zu einer Lösung von 35 g 2-Chlorbenzaldehyd in 300 ml Methanol werden 4,2 g Natriumhydroxid in 30 ml Wasser gegeben. Das Reaktionsgemisch wird auf 10 °C gekühlt und schnell 36 g 4-Fluorphenylacetaldehyd zugesetzt, wobei die Temperatur in der Lösung auf 30-40 °C ansteigt. Nach 10-stündigem Rühren bei 40 °C werden die ausgefallenden Kristalle aus der abgekühlten Reaktionslösung abgesaugt.

Vorschrift 2

cis-2-Formyl-2-(4-fluorphenyl)-3-(2-chlorphenyl)oxiran

78,2 g E-2-(4-Fluorphenyl)-3-(2-chlorphenyl)-propenal werden in 300 ml Methanol gelöst und 1 ml Natronlauge (konz.) zugesetzt. Die Reaktionslösung wird bei 0 °C gerührt, während 20,5 g Wasserstoffperoxyd (ca. 50 %ig) langsam zugetropft werden, wobei die Innentemperatur von 30 °C nicht überschritten wird. Nach beendeter Zugabe wird sechs Stunden bei Raumtemperatur gerührt, anschließend 100 ml Wasser zugesetzt und die entstandene Emulsion mit Methyl-tert.-butylether ausgeschüttelt. Die isolierte organische Phase wird daraufhin über Natriumsulfat getrocknet und eingeengt. Man erhält 52,5 g (63 %) cis-2-Formyl-2-(4-fluorphenyl)-3-(2-chlorphenyl)-oxiran.

Vorschrift 3

1'RS-cis-2-[1-(1,2,4-Triazol-1-yl)-1-chlor-methyl]-2-(4-fluor-phenyl)-3-(2-chlorphenyl)-oxiran

Zu einer Lösung von 29,7 g Triazol in 150 ml Methylenchlorid wird bei 0 °C unter Stickstoffatmosphäre 12,8 g Thionylchlorid zugesetzt. Nach beendeter Zugabe wird bei Raumtemperatur für 30 Minuten gerührt und anschließend 20 g cis-2-Formyl-2-(4-fluorphenyl)-3-(2-chlorphenyl)-oxiran zugegeben. Nachdem das Reaktionsgemisch 12-15 Stunden bei Raumtemperatur rührte, wird der Lösung 100 ml Wasser zugesetzt und die organische Phase abgetrennt. Die verbleibende wässrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet und eingeengt, wobei 23,7 g (85%) cis-2-[1-(1,2,4-Triazol-1-yl)-1-chlor-methyl]-2-(4-fluorphenyl)-3-(2-chlorphenyl)-oxiran als 2:1-Diastereomerengemisch erhalten werden. Man erhält aus Methyl-tert.-butylether 5,8 g des vermehrt gebildeten Diastereomeren A mit dem Schmelzpunkt 152-156 °C.

II. Herstellung der Endprodukte

Beispiel 1

5 g 1'RS-cis-2-[1-(1,2,4-triazol-1-yl)-1-chlor-methyl]-2-(4-fluor-phenyl)-3-(2-chlorphenyl)-oxiran werden in 100 ml Methanol gelöst und mit 1,7 g Natriummethylat versetzt und für eine Stunde unter Rückfluß erhitzt. Anschließend wird die Lösung bei Raumtemperatur mit 100 ml Wasser versetzt und mehrmals mit Methyl-tert.-butylether ausgeschüttelt. Die isolierte organische Phase wird zweimal mit Wasser gewaschen, daraufhin über Natriumsulfat getrocknet und eingeengt, wobei 4,4 g (87 %) 1-Chlor-1-(1,2,4-triazol-1-yl)-2-(4-fluorphenyl)-3-(2-chlor-phenyl)-propen -3-ol (Verbindung Nr. 1) erhalten werden, Fp.: 153-156 °C.

Entsprechend Beispiel 1 können die in der Tabelle aufgeführten Verbindungen hergestellt werden.

Tabelle

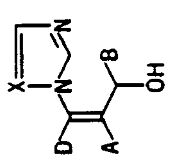

| Bsp | A | B | D | X | Schmp./IR ($cm^{-1}$) |
|---|---|---|---|---|---|
| 1 | $4-F-C_6H_4$ | $2-Cl-C_6H_4$ | Cl | N | 153-156°C |
| 2 | $4-F-C_6H_4$ | $2-Cl-C_6H_4$ | Br | N | 1503, 1413, 1213, 1126, 853, 752 |
| 3 | $4-F-C_6H_4$ | $2-Cl-C_6H_4$ | Cl | CH | 1509, 1228, 1069, 1057, 756 |
| 4 | $4-F-C_6H_4$ | $2-Cl-C_6H_4$ | Br | CH | |
| 5 | $4-F-C_6H_4$ | $4-Cl-C_6H_4$ | Cl | N | |
| 6 | $4-F-C_6H_4$ | $4-Cl-C_6H_4$ | Cl | CH | |
| 7 | $4-F-C_6H_4$ | $2,4-Cl_2-C_6H_3$ | Cl | N | |
| 8 | $4-F-C_6H_4$ | $2,4-Cl_2-C_6H_3$ | Cl | CH | |
| 9 | $4-F-C_6H_4$ | $2,4-Cl_2-C_6H_3$ | Br | N | |
| 10 | $4-F-C_6H_4$ | $C_6H_5$ | Cl | N | |
| 11 | $4-F-C_6H_4$ | $C_6H_5$ | Br | N | |
| 12 | $4-F-C_6H_4$ | $2-Cl-4-F-C_6H_3$ | Cl | N | |
| 13 | $4-F-C_6H_4$ | $2-F-C_6H_4$ | Cl | N | |
| 14 | $4-F-C_6H_4$ | $2-F-C_6H_4$ | Br | N | |
| 15 | $4-F-C_6H_4$ | $2-F-C_6H_4$ | Cl | CH | |
| 16 | $4-F-C_6H_4$ | $4-F-C_6H_4$ | Cl | N | |
| 17 | $4-F-C_6H_4$ | $4-F-C_6H_4$ | Br | N | |
| 18 | $4-F-C_6H_4$ | $2,4-F_2-C_6H_3$ | Cl | N | |

Tabelle (Fortsetzung)

| Bsp | A | B | D | X | Schmp./IR ($cm^{-1}$) |
|---|---|---|---|---|---|
| 19 | $4-F-C_6H_4$ | $2,4-F_2-C_6H_3$ | Cl | CH | 1617, 1603, 1507, 1502, 1229, 1096, 964, 852 |
| 20 | $4-F-C_6H_4$ | $2,4-F_2-C_6H_3$ | Br | N | |
| 21 | $4-F-C_6H_4$ | $2-Br-C_6H_4$ | Cl | N | 160-169°C |
| 22 | $4-F-C_6H_4$ | $2-Br-C_6H_4$ | Cl | CH | |
| 23 | $4-F-C_6H_4$ | $2-Br-C_6H_4$ | Br | N | |
| 24 | $4-F-C_6H_4$ | $2-CF_3-C_6H_4$ | Cl | N | 1508, 1163, 1123, 771 |
| 25 | $4-F-C_6H_4$ | $2-CF_3-C_6H_4$ | Cl | CH | |
| 26 | $4-F-C_6H_4$ | $2-CF_3-C_6H_4$ | Br | N | |
| 27 | $4-F-C_6H_4$ | $2-CF_3-C_6H_4$ | Br | CH | |
| 28 | $4-F-C_6H_4$ | $4-CF_3-C_6H_4$ | Cl | N | |
| 29 | $4-F-C_6H_4$ | $4-CF_3-C_6H_4$ | Cl | CH | 1510, 1326, 1126, 1067, 838 |
| 30 | $4-F-C_6H_4$ | $4-NO_2-C_6H_4$ | Cl | N | |
| 31 | $4-F-C_6H_4$ | $4-NH_2-C_6H_4$ | Cl | N | |
| 32 | $4-F-C_6H_4$ | $2-CH_3-C_6H_4$ | Cl | N | |
| 33 | $4-F-C_6H_4$ | $2-CH_3-C_6H_4$ | Cl | N | |
| 34 | $4-F-C_6H_4$ | $4-C_6H_5O-C_6H_4$ | Cl | N | |
| 35 | $4-F-C_6H_4$ | $2-OCH_3-C_6H_4$ | Cl | N | 1603, 1504, 1241, 1048, 756 |
| 36 | $4-F-C_6H_4$ | $2-OCH_3-C_6H_4$ | Cl | CH | |
| 37 | $4-F-C_6H_4$ | $2-OCH_3-C_6H_4$ | Br | N | |
| 38 | $4-F-C_6H_4$ | 1-Naphthyl | Cl | N | |
| 39 | $4-F-C_6H_4$ | 2-Naphthyl | Cl | N | |
| 40 | $4-F-C_6H_4$ | 2-Naphthyl | Cl | CH | |

Tabelle (Fortsetzung)

| Bsp | A | B | D | X | Schmp./IR ($cm^{-1}$) |
|---|---|---|---|---|---|
| 41 | 4-F-$C_6H_4$ | 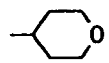 | Cl | N | |
| 42 | 4-F-$C_6H_4$ | Cyclopropyl | Cl | N | |
| 43 | 4-F-$C_6H_4$ | Cyclopentyl | Cl | N | |
| 44 | 4-F-$C_6H_4$ | Cyclohexyl | Cl | N | |
| 45 | 4-F-$C_6H_4$ | 3-Cyclohexenyl | Cl | N | |
| 46 | $C_6H_5$ | $C_6H_5$ | Cl | N | |
| 47 | $C_6H_5$ | 2-Cl-$C_6H_4$ | Cl | N | |
| 48 | $C_6H_5$ | 2-Cl-$C_6H_4$ | Cl | CH | |
| 49 | $C_6H_5$ | 2-Cl-$C_6H_4$ | Br | N | |
| 50 | $C_6H_5$ | 4-Cl-$C_6H_4$ | Cl | N | Harz |
| 51 | $C_6H_5$ | 4-Cl-$C_6H_4$ | Cl | CH | Harz |
| 52 | $C_6H_5$ | 2,4-$Cl_2$-$C_6H_3$ | Cl | N | 1506, 1276, 1133, 1100, 806, 701 $cm^{-1}$ |
| 53 | $C_6H_5$ | 2,4-$Cl_2$-$C_6H_3$ | Cl | CH | |
| 54 | $C_6H_5$ | 2-F-$C_6H_4$ | Cl | N | |
| 55 | $C_6H_5$ | 4-F-$C_6H_4$ | Cl | N | |
| 56 | $C_6H_5$ | 2-$CF_3$-$C_6H_4$ | Cl | N | |
| 57 | $C_6H_5$ | 2-$CF_3$-$C_6H_4$ | Cl | CH | |
| 58 | $C_6H_5$ | 4-$CF_3$-$C_6H_4$ | Cl | N | |
| 59 | $C_6H_5$ | 4-$CF_3$-$C_6H_4$ | Cl | CH | |
| 60 | $C_6H_5$ | 2-Br-$C_6H_4$ | Cl | N | |
| 61 | $C_6H_5$ | 2-Br-$C_6H_4$ | Cl | CH | |
| 62 | 4-Cl-$C_6H_4$ | $C_6H_5$ | Cl | N | |
| 63 | 4-Cl-$C_6H_4$ | 2-Cl-$C_6H_4$ | Cl | N | |

Tabelle (Fortsetzung)

| Bsp | A | B | D | X | Schmp./IR ($cm^{-1}$) |
|---|---|---|---|---|---|
| 64 | 4-Cl-$C_6H_4$ | 2-Cl-$C_6H_4$ | Cl | CH | |
| 65 | 4-Cl-$C_6H_4$ | 4-Cl-$C_6H_4$ | Cl | N | |
| 66 | 4-Cl-$C_6H_4$ | 4-Cl-$C_6H_4$ | Cl | CH | |
| 67 | 4-Cl-$C_6H_4$ | 2,4-$Cl_2$-$C_6H_3$ | Cl | N | |
| 68 | 4-Cl-$C_6H_4$ | 2,4-$Cl_2$-$C_6H_3$ | Cl | CH | |
| 69 | 4-Cl-$C_6H_4$ | 2-F-$C_6H_4$ | Cl | N | |
| 70 | 4-Cl-$C_6H_4$ | 2-F-$C_6H_4$ | Cl | CH | |
| 71 | 4-Cl-$C_6H_4$ | 4-F-$C_6H_4$ | Cl | N | |
| 72 | 4-Cl-$C_6H_4$ | 4-F-$C_6H_4$ | Cl | CH | |
| 73 | 4-Cl-$C_6H_4$ | 2-$CF_3$-$C_6H_4$ | Cl | N | |
| 74 | 4-Cl-$C_6H_4$ | 2-$CF_3$-$C_6H_4$ | Cl | CH | |
| 75 | 4-Cl-$C_6H_4$ | 4-$CF_3$-$C_6H_4$ | Cl | N | |
| 76 | 2-Cl-$C_6H_4$ | $C_6H_5$ | Cl | N | |
| 77 | 2-Cl-$C_6H_4$ | 2-Cl-$C_6H_4$ | Cl | N | |
| 78 | 2-Cl-$C_6H_4$ | 4-Cl-$C_6H_4$ | Cl | N | |
| 79 | 2-Cl-$C_6H_4$ | 2,4-$Cl_2$-$C_6H_3$ | Cl | N | |
| 80 | 2-Cl-$C_6H_4$ | 2-F-$C_6H_4$ | Cl | N | |
| 81 | 2-Cl-$C_6H_4$ | 4-F-$C_6H_4$ | Cl | N | |
| 82 | 2-Cl-$C_6H_4$ | 2-$CF_3$-$C_6H_4$ | Cl | N | |
| 83 | 2-F-$C_6H_4$ | $C_6H_5$ | Cl | N | |
| 84 | 2-F-$C_6H_4$ | 2-Cl-$C_6H_4$ | Cl | N | |
| 85 | 2-F-$C_6H_4$ | 4-Cl-$C_6H_4$ | Cl | N | |
| 86 | 2,4-$Cl_2$-$C_6H_3$ | 2-Cl-$C_6H_4$ | Cl | N | |
| 87 | 2,4-$Cl_2$-$C_6H_3$ | 4-Cl-$C_6H_4$ | Cl | N | |
| 88 | 2,4-$Cl_2$-$C_6H_3$ | 4-F-$C_6H_4$ | Cl | N | |

Tabelle (Fortsetzung)

| Bsp | A | B | D | X | Schmp./IR ($cm^{-1}$) |
|---|---|---|---|---|---|
| 89 | p-Biphenyl | $C_6H_5$ | Cl | N | |
| 90 | Cyclohexyl | $C_6H_5$ | Cl | N | |
| 91 | Cyclohexyl | 2-Cl-$C_6H_4$ | Cl | N | |
| 92 | Cyclohexyl | 4-Cl-$C_6H_4$ | Cl | N | |
| 93 | Cyclohexyl | 2,4-$Cl_2$-$C_6H_3$ | Cl | N | |
| 94 | Cyclohexyl | 2,4-$Cl_2$-$C_6H_3$ | Cl | CH | |
| 95 | Cyclohexyl | 2-Br-$C_6H_4$ | Cl | N | |
| 96 | Cyclohexyl | 2-Br-$C_6H_4$ | Cl | CH | |
| 97 | Cyclohexyl | 2-F-$C_6H_4$ | Cl | N | |
| 98 | Cyclohexyl | 4-F-$C_6H_4$ | Cl | N | |
| 99 | Cyclohexyl | 4-F-$C_6H_4$ | Br | N | |
| 100 | Cyclohexyl | 2-$CF_3$-$C_6H_4$ | Cl | N | |
| 101 | Cyclohexyl | 4-$CF_3$-$C_6H_4$ | Cl | N | |
| 102 | Cyclohexyl | 3-Pyridyl | Cl | N | |
| 103 | tert.-$C_4H_9$ | $C_6H_5$ | Cl | N | |
| 104 | tert.-$C_4H_9$ | 2-Cl-$C_6H_4$ | Cl | N | |
| 105 | tert.-$C_4H_9$ | 4-Cl-$C_6H_4$ | Cl | N | |
| 106 | tert.-$C_4H_9$ | 2,4-$Cl_2$-$C_6H_3$ | Cl | N | |
| 107 | tert.-$C_4H_9$ | 2-Br-$C_6H_4$ | Cl | N | |
| 108 | tert.-$C_4H_9$ | 2-F-$C_6H_4$ | Cl | N | |
| 109 | tert.-$C_4H_9$ | 4-F-$C_6H_4$ | Cl | N | |
| 110 | tert.-$C_4H_9$ | 2-$CF_3$-$C_6H_4$ | Cl | N | |
| 111 | tert.-$C_4H_9$ | 4-$CF_3$-$C_6H_4$ | Cl | N | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,

Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Verbindungen können praktisch alle Entwicklungstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, werden einige nachstehend erwähnt.

A.

Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Reis, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt.

Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- und Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen die dichtere Bepflanzung des Bodens, so daß ein Mehrertrag, bezogen auf die Bodenfläche, erzielt werden kann.

B.

Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C.

Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftichem Interesse ist beispielsweise die Ernteerleicherung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wesentlich.

D.

Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite des Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendeten Wirkstoffe können den Kulturpflanzen sowohl vom Samen her (als Saatgutbeizmittel) als auch über den Boden, d.h. durch die Wurzel sowie über das Blatt zugeführt werden.

Infolge der hohen Pflanzenverträglichkeit kann die Aufwandmenge als Wachstumsregulatoren stark variiert werden.

Bei der Saatgutbehandlung werden im allgemeinen Wirkstoffmengen von 0,001 bis 50 g, vorzugsweise 0,01 bis 10 g je Kilogramm Saatgut benötigt.

Für die Blatt- und Bodenbehandlung sind im allgemeinen Gaben von 0,01 bis 10 kg/ha, bevorzugt 0,02 bis 3 kg/ha, als ausreichend zu betrachten.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 24 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 19 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 29 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 24 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 19 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

## Anwendungsbeispiele

Als Vergleichssubstanzen dienten die Verbindungen 1-(1,2,4-Triazol-1-yl)-2-(4-chlorphenyl)-3-phenyl-propen-1-ol-3 (A) und 1-(1,2,4-Triazol-1-yl)-2-(4-chlorphenyl)-3-(2,4-dichlorphenyl)-propen-1-ol-3 (B) - bekannt aus EP 23 286.

### Anwendungsbeispiel 1

### Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24 °C und 95 bis 99 % relativer Luftfeuchtigkeit aufgestellt. Nach 6 Tagen wurde das Ausmaß des Krankheitsbefalls ermittelt.

Das Ergebnis zeigt, daß die Wirkstoffe 1, 2, 19 und 29 bei der Anwendung als 0,05 %ige (Gew.%) Spritzbrühe eine bessere fungizide Wirkung zeigen (90 %) als der bekannte Vergleichswirkstoff B (40 %).

Anwendungsbeispiel 2

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22 °C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22 °C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 1, 2, 3 und 24 bei der Anwendung als 0,006 %ige (Gew.-%) Spritzbrühe eine bessere fungizide Wirkung zeigen (95 %) als der bekannte Vergleichswirkstoff B (40 %).

Anwendungsbeispiel 3

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt. Anschließend wurden die Pflanzen im Gewächshaus bei 20 bis 22 °C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 2, 3, 19, 29 und 51 bei der Anwendung als 0,05 %ige Spritzbrühe eine bessere fungizide Wirkung zeigen (90 %) als die bekannten Vergleichwirkstoffe A und B (0 %).

Zur Bestimmung der wachstumsregulierenden Eigenschaften der Prüfsubstanzen wurden die Testpflanzen auf ausreichend mit Nährstoffen versorgtem Kultursubstrat in Kunststoffgefäßen (ca. 12,5 cm Durchmesser; Volumen ca. 500 ml) angezogen.

Im Vorauflaufverfahren wurden die Testsubstanzen in wäßriger Aufbereitung am Tage der Einsaat auf das Saatbett gegossen. Im Nachauflaufverfahren wurden die zu prüfenden Substanzen in wäßriger Aufbereitung auf die Pflanzen gesprüht.

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Anwendungsbeispiel 4

Tabelle 1: Sommerweizen, "Ralle"

Nachauflauf-Blattbehandlung

| Wirkstoff | Konz. mg Wirkstoff/Gefäß | Wuchshöhen relativ in % |
|---|---|---|
| unbehandelt | - | 100 |
| A | 6 | 100 |
| 1 | 6 | 92,8 |
| 19 | 6 | 94,4 |
| B | 6 | 100 |
| 50 | 6 | 88,9 |

Tabelle 2: Sommergerste, "Aramir"

Vorauflauf-Bodenbehandlung

| Wirkstoff | Konz. mg Wirkstoff/Gefäß | Wuchshöhen relativ in % |
|---|---|---|
| unbehandelt | - | 100 |
| A | 6 | 100 |
| 1 | 6 | 93,9 |

Tabelle 3: Sommergerste, "Aramir"

Nachauflauf-Blattbehandlung

| Wirkstoff | Konz. mg Wirkstoff/Gefäß | Wuchshöhen relativ in % |
|---|---|---|
| unbehandelt | - | 100 |
| A | 6 | 100 |
| 19 | 6 | 90,2 |

Tabelle 4: Sonnenblumen, "Spanners Allzweck"

Nachauflauf-Blattbehandlung

| Wirkstoff | Konz. mg Wirkstoff/Gefäß | Wuchshöhen relativ in % |
|---|---|---|
| unbehandelt | - | 100 |
| B | 6 | 100 |
| 29 | 6 | 86,4 |

## Patentansprüche

**1.** 1-Halogenvinyl-azole der allgemeinen Formel I

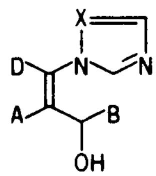

I

in welcher A und B $C_1$-$C_8$-Alkyl, $C_5$-$C_8$-Cycloalkyl, $C_5$-$C_8$-Cycloalkenyl, Pyridyl, Tetrahydropyranyl, Naphthyl, Biphenyl oder Phenyl bedeuten, wobei diese Reste durch Halogen, Nitro, Phenoxy, Amino, $C_1$-$C_4$-Alkyl, $C_1$-$C_4$-Alkoxy oder Halogen-$C_1$-$C_4$-alkyl substituiert sein können,
D den Rest Chlor oder Brom bedeutet,
X den Rest CH oder N bedeutet,
sowie deren für Pflanzen verträgliche Säureadditionssalze und Metallkomplexe.

**2.** 1-Halogen-Vinylazole der allgemeinen Formel I, in denen A und B die Phenylgruppe bedeutet, welche unsubstituiert oder durch einen oder zwei Substituenten substituiert ist, die Fluor, Chlor, Brom oder die Trifluormethylgruppe bedeuten.

**3.** Verfahren zur Herstellung der 1-Halogen-Vinylazole der Formel I gemäß Anspruch 1, dadurch gekennzeichnet, daß man eine Verbindung der Formel II

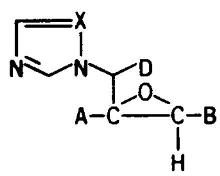

II

in welcher A,B,D und X die in Anspruch 1 angegebene Bedeutung haben, in Gegenwart einer Base in die 1-Halogen-Vinylazole umlagert.

**4.** Fungizides Mittel, enthaltend einen Trägerstoff und eine fungizid wirksame Menge eines 1-Halogen-1-Vinylazols der Formel I gemäß Anspruch 1.

**5.** Verfahren zur Bekämpfung von Pilzen außerhalb des menschlichen Körpers, dadurch gekennzeichnet, daß man eine fungizid wirksame Menge eines 1-Halogen-Vinylazols der Formel I gemäß Anspruch 1 auf die Pilze oder durch Pilzbefall bedrohte Materialien, Flächen, Pflanzen oder Saatgüter einwirken läßt.

**6.** Wachstumsregulierendes Mittel, enthaltend einen Trägerstoff und ein 1-Halogen-Vinylazol der Formel I gemäß Anspruch 1.

**7.** Verfahren zur Regulierung des Pflanzenwachstums, dadurch gekennzeichnet, daß man eine regulierend wirksame Menge eines 1-Halogen-Vinylazols der Formel I gemäß Anspruch 1 auf Kulturpflanzen oder den Boden einwirken läßt.

**8.** Verbindung der Formel I gemäß Anspruch 1, dadurch gekennzeichnet, daß A 4-Fluorphenyl, B 2-Chlorphenyl, D Chlor und X N bedeutet.

**9.** Verbindung der Formel I gemäß Anspruch 1, dadurch gekennzeichnet, daß A 4-Fluorphenyl, B 2-Chlorphenyl, D Brom und X N bedeutet.

**10.** Verbindung der Formel I gemäß Anspruch 1, dadurch gekennzeichnet, daß A 4-Fluorphenyl, B 2-Chlorphenyl, D Chlor und X CH bedeutet.

## Claims

**1.** A 1-halovinylazole of the general formula I

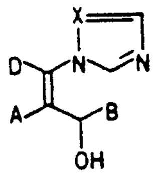

I

where A and B are each $C_1$-$C_8$-alkyl, $C_5$-$C_8$-cycloalkyl, $C_5$-$C_8$-cycloalkenyl, pyridyl, tetrahydropyranyl, naphthyl, biphenylyl or phenyl, it being possible for each of these radicals to be substituted by halogen, nitro, phenoxy, amino, $C_1$-$C_4$-alkyl, $C_1$-$C_4$-alkoxy or halo-$C_1$-$C_4$-alkyl,
D is chlorine or bromine,
X is CH or N,
or an acid addition salt or metal complex thereof which is tolerated by plants.

**2.** A 1-halovinylazole of the general formula I where A and B are phenyl which is unsubstituted or substituted by one or two substituents which are fluorine, chlorine, bromine or trifluoromethyl.

**3.** A process for preparing a 1-halovinylazole of the formula I as claimed in claim 1, which comprises rearranging a compound of the formula II

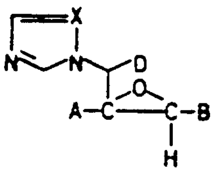

II

where A, B, D and X have the meanings specified in claim 1, in the presence of a base, to give a 1-halovinylazole.

**4.** A fungicide containing a carrier and a fungicidally effective amount of a 1-halo-1-vinylazole of the formula I as claimed in claim 1.

**5.** A method of controlling fungi outside of the human body, wherein a fungicidally effective amount of a 1-halovinylazole of the formula I as claimed in claim 1 is allowed to act on the fungi, or on the materials, areas, plants or seed threatened by fungal attack.

**6.** A growth regulator containing a carrier and a 1-halovinylazole of the formula I as claimed in claim 1.

**7.** A method of regulating plant growth, wherein a regulating amount of a 1-halovinylazole of the formula I as claimed in claim 1 is allowed to act on crops or the soil.

**8.** A compound of the formula I as claimed in claim 1, wherein A is 4-fluorophenyl, B is 2-chlorophenyl, D is chlorine and X is N.

**9.** A compound of the formula I as claimed in claim 1, wherein A is 4-fluorophenyl, B is 2-chlorophenyl, D is bromine and X is N.

**10.** A compound of the formula I as claimed in claim 1, wherein A is 4-fluorophenyl, B is 2-chlorophenyl, D is chlorine and X is CH.

## Revendications

1. 1-halogène-vinyl-azoles de formule générale I

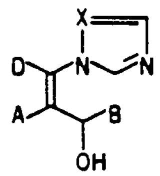

I

dans laquelle A et B représentent alkyle en C1-C8, cycloalkyle en C5-C8, cycloalcényle en C5-C8, pyridyle, tétrahydropyranyle, naphtyle, biphényle ou phényle, ces restes pouvant être substitués par halogène, nitro, phénoxy, amino, alkyle en C1-C4, alcoxy en C1-C4 ou halogène-alkyle en C1-C4,
D représente le reste chlore ou brome,
X représente le reste CH ou N,
ainsi que leurs sels d'addition d'acide et complexes métalliques tolérés par les plantes.

2. 1-halogène-vinylazoles de formule générale I, dans lesquels A et B représentent le groupe phényle, qui est non substitué ou substitué par un ou deux substituants, à savoir fluor, chlore, brome ou le groupe trifluorométhyle.

3. Procédé de préparation des 1-halogène-vinylazoles de formule I, selon la revendication 1, caractérisé par le fait que l'on transforme, en présence d'une base, un composé de formule

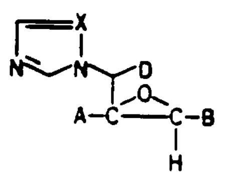

II

dans laquelle A, B, D et X ont les significations données dans la revendication 1, en 1-halogène-vinylazole.

4. Agent fongicide, contenant un support et une quantité efficace au point de vue fongicide d'un 1-halogène-1-vinylazole de formule I, selon la revendication 1.

5. Procédé de lutte contre des champignons à l'extérieur du corps humain, caractérisé par le fait que l'on fait agir une quantité efficace au point de vue fongicide d'un 1-halogène-vinylazole de formule I, selon la revendication 1, sur les champignons ou les matériaux, surfaces, plantes ou semences menacés par l'attaque par les champignons.

6. Agent régularisant la croissance, contenant un support et un 1-halogène-vinylazole de formule I, selon la revendication 1.

7. Procédé pour régulariser la croissance des plantes, caractérisé par le fait que l'on fait agir sur les plantes cultivées ou le sol un 1-halogène-vinylazole de formule I, selon la revendication 1, en quantité efficace pour régulariser la croissance.

8. Composé de formule I, selon la revendication 1, caractérisé par le fait que A représente 4-fluorophényle, B 2-chlorophényle, D chlore et X N.

9. Composé de formule I, selon la revendication 1, caractérisé par le fait que A représente 4-fluorophényle, B 2-chlorophényle, D brome et X N.

10. Composé de formule I, selon la revendication 1, caractérisé par le fait que A représente fluorophényle, B 2-chlorophényle, D chlore et X CH.